# FASCICULE DE BREVET EUROPEEN

(11) **EP 3 391 262 B1**
(45) Date de publication et mention de la délivrance du brevet: **14.05.2025**
(21) Numéro de dépôt: 16825361.5
(22) Date de dépôt: 16.12.2016
(51) Int. Cl.: G16B 50/30, G16B 50/40

(54) **ARCHITECTURE POUR L'ANALYSE DE DONNEES GENOMIQUES**
ARCHITEKTUR ZUR ANALYSE VON GENOMDATEN
ARCHITECTURE FOR ANALYZING GENOMIC DATA

(30) Priorité: 18.12.2015 FR 1562828
(43) Date de publication de la demande: 24.10.2018
(73) Titulaire: Seqone, 34000 Montpellier (FR)
(72) Inventeur: PHILIPPE, Nicolas, 34800 Clermont l'hérault (FR); BUWALDA, Guillaume, 92300 Levallois (FR)
(74) Mandataire: Icosa
(86) Numéro de dépôt international: PCT/EP2016/081575
(87) Numéro de publication internationale: WO 2017/103202

(56) Documents cités:
- US-A1- 2010 281 401
- US-A1- 2015 310 228

## Description

### DOMAINE TECHNIQUE DE L'INVENTION

Un aspect de la présente invention porte sur une architecture pour l'analyse de données génomiques, telles que des courtes séquences d'acide ribonucléique (ARN) ou d'acide désoxyribonucléique (ADN).

### ARRIERE-PLAN TECHNOLOGIQUE DE L'INVENTION

Les séquenceurs d'ADN sont apparus dans les années 1970 avec la mission de numériser l'ADN des cellules d'organismes vivants. Ces séquenceurs ont notamment permis le séquençage du premier génome humain au cours des années 1990. À cette époque, ce séquençage a requis plus de 10 ans de travaux et environ 1 milliard de dollars. Récemment, une avancée technologique majeure s'est traduite par l'émergence d'une nouvelle génération de séquenceurs à très haut débit (également connu sous l'acronyme NGS), et a modifié le paysage de la biologie cellulaire et moléculaire. Le prix du séquençage est devenu très accessible et l'augmentation vertigineuse de la capacité de production de ces séquenceurs a permis le séquençage des génomes entiers en seulement quelques jours.

Aujourd'hui, cette révolution technologique bouleverse la biologie et la médecine. En cancérologie par exemple, il est désormais possible de séquencer très rapidement l'ensemble des informations contenues dans les cellules cancéreuses d'un patient, dans l'optique de rechercher des marqueurs moléculaires qui serviront pour le diagnostic, le pronostic, le suivi de la maladie résiduelle ou la réponse au traitement. Cependant, les séquenceurs ont toujours été limités, et continuent de l'être, par la taille des fragments d'ADN séquencés. La préparation d'un échantillon consiste à le découper en courts brins d'ADN (de quelques centaines de nucléotides seulement) pour qu'un séquenceur puisse produire, très rapidement, des centaines de millions de courtes séquences d'ADN (ou d'ARN), sous une forme textuelle : on parle de reads. Ces reads couvrent entièrement le matériel génétique de départ (par exemple le génome ou le transcriptome d'un individu). Mais, l'information qu'ils produisent est découpée en un puzzle de millions de lectures complètement désorganisées et dont certaines contiennent des erreurs produites lors du séquençage ou de la préparation de l'échantillon. Autrement dit, les séquences produites par les NGS sont contraintes et n'aboutissent pas à des résultats exploitables simplement.

Biologistes et cliniciens sont confrontés à un déluge de données (Big Data) qu'il faut stocker, trier, structurer et interpréter. Néanmoins, aucun outil adapté à l'exploitation de ces données n'est aujourd'hui proposé aux biologistes ni même aux cliniciens.

### DESCRIPTION GENERALE DE L'INVENTION

Dans ce contexte, la présente invention vise à fournir une architecture pour l'analyse de données génomiques permettant d'exploiter efficacement les données de génomiques obtenues au moyen d'un séquenceur à très haut débit.

A cette fin, l'invention porte sur une architecture pour l'analyse de données génomiques comme définie dans la revendication indépendante 1.

Grâce à l'invention, un utilisateur peut effectuer, via un système de calcul, des calculs sur des données de génomiques contenues par exemple dans la base de connaissances. Les résultats des calculs réalisés peuvent être stockés dans la base de connaissances et être accessibles à d'autres utilisateurs, lesquels autres utilisateurs peuvent par exemple enrichir ces résultats en les comparant avec les leurs.

Cette architecture forme donc un environnement de travail collaboratif pour les analyses de données issues des nouvelles technologies de séquençage à très haute résolution (NGS). Cet environnement de travail collaboratif permet de réaliser des analyses génomiques précises et interprétées d'une pathologie ou d'un patient, en lien avec une question biologique et/ou médicale, tout en offrant un accès simplifié, et en toute autonomie, à des résultats complexes.

En outre, il convient de noter que l'architecture repose sur :
- un système multi-tenants car plusieurs utilisateurs peuvent utiliser un seul nœud de calcul d'un unique système de calcul, et
- un système multi-instances car un utilisateur peut utiliser plusieurs nœuds de calcul d'un système de calcul.

Cette architecture hybride permet de tirer le meilleur parti des deux systèmes et ainsi garantir une confidentialité des utilisateurs les plus consommateurs en ressources de calculs.

Dans une mise en œuvre non limitative, l'architecture comporte une pluralité d'espaces partagés, chacun des espaces partagés étant construit et agencé :
- pour contenir des métadonnées et des résultats issus de calculs collaboratifs réalisés par les systèmes de calcul d'une entité authentifiée comportant 1 à n utilisateur, et
- être accessible en lecture uniquement aux utilisateurs des entités authentifiées et bénéficiant d'un droit de consultation audit espace.

Dans une mise en œuvre non limitative, l'architecture comporte un espace commun, ledit espace commun étant accessible à tous les utilisateurs des entités authentifiées de l'architecture.

Dans une mise en œuvre non limitative, le système de calcul est construit et agencé pour classer des courtes séquences numériques de matériel biologique.

Dans une mise en œuvre non limitative, le système de calcul est construit et agencé pour agréger les courtes séquences numériques de matériel biologique d'une même classe.

Dans une mise en œuvre non limitative, le système de calcul est construit et agencé pour identifier une séquence biomarqueur répondant à une question biologique.

Dans une mise en œuvre non limitative, l'architecture comporte une pluralité de ressources, lesdites ressources étant formées par :
- Un système d'identification d'une entité et des éléments qu'elle manipule,
- Un système d'identification d'un utilisateur,
- Un système d'identification d'un calcul associé à au moins un utilisateur et une entité,
- Un système permettant de corréler des calculs réalisés,
- Un système de regroupement logique d'identifiants de calculs associés à un utilisateur et une entité,
- Un système permettant d'associer une méta-information à des données de la base de connaissances.

Dans une mise en œuvre non limitative, la base de connaissances est formée par un anneau de base de données.

Dans une mise en œuvre non limitative, l'interface d'accès à la base de connaissances est une API REST.

### BREVE DESCRIPTION DE LA FIGURE

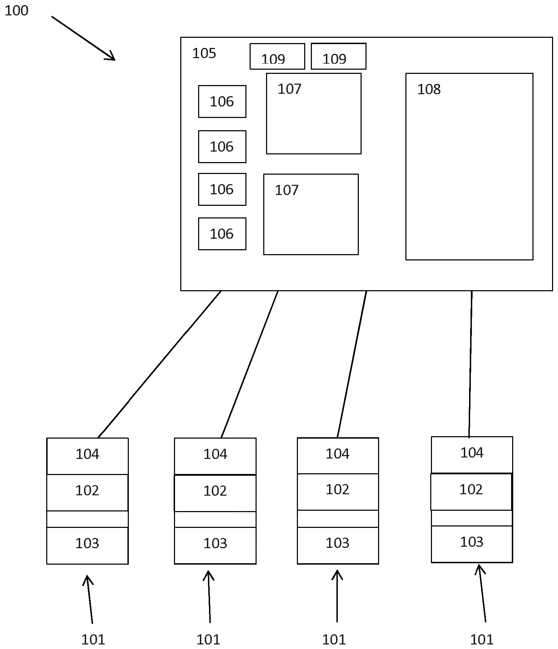

D'autres caractéristiques et avantages de l'invention ressortiront clairement de la description qui en est donnée ci-dessous, à titre indicatif et nullement limitatif, en référence à la figure 1 annexée illustrant, de façon schématique.

### DESCRIPTION DETAILLEE D'AU MOINS UN MODE DE REALISATION DE L'INVENTION

La figure 1 illustre un exemple de réalisation d'une architecture 100 pour l'analyse de données génomiques selon un aspect non limitatif de l'invention.

Cette architecture 100 comporte une pluralité de nœuds applicatifs 101. Chacun des nœuds applicatifs 101 comporte un système de calcul 102 comportant au moins un nœud de calcul, le système de calcul 102 étant construit et agencé pour réaliser des calculs de génomique.

Lorsque le système de calcul 102 est sollicité par un utilisateur en vue de réaliser un calcul nécessitant des ressources importantes, le système de calcul 102 peut implémenter plusieurs nœuds de calcul. On rappelle que lorsque l'on prête une action à un nœud de calcul celle-ci est en fait effectuée par un microprocesseur du nœud de calcul commandé par des codes instructions enregistrés dans une mémoire du nœud de calcul. Si l'on prête une action à une application, celle-ci est en fait effectuée par un microprocesseur du nœud de calcul dans une mémoire duquel les codes instructions correspondant à l'application sont chargés.

Pour réaliser ces calculs de génomique, le système de calcul 102 est construit et agencé pour charger des outils bio-informatiques permettant d'organiser et d'interpréter des séquences biomarqueurs. En d'autres termes, on entend par calcul notamment le fait d'organiser et d'interpréter des séquences biomarqueurs. Les outils bio-informatiques pouvant être chargés par le système de calcul 102 peuvent par exemple être formés par différents type de logiciels tels que le logiciel CRAC, le logiciel CracTools, ou encore la suite logicielle CRAC & the CT.

Dans une mise en œuvre non limitative, le système de calcul 102 peut être construit et agencé pour classer des courtes séquences numériques de matériel biologique. Pour ce faire, le système de calcul 102 peut par exemple implémenter le logiciel dénommé CRAC qui est spécialisé dans le traitement des séquences RNA-Seq et permet de classer les reads. Les RNA-Seq sont obtenus par le séquençage à très haut débit des ARN.

Dans une mise en œuvre non limitative, le système de calcul 102 est en outre construit et agencé pour agréger des courtes séquences numériques de matériel biologique d'une même classe. Pour ce faire, le système de calcul 102 peut par exemple implémenter le logiciel dénommé CracTools qui est spécialisé dans le post-traitement des reads classés au moyen du logiciel CRAC et permet d'agréger les reads d'une même classe pour identifier une séquence biomarqueur.

Dans une autre mise en œuvre non limitative, le système de calcul 102 est en outre construit et agencé pour identifier une séquence biomarqueur répondant à une question biologique. Pour ce faire, le système de calcul 102 peut par exemple implémenter la suite logicielle dénommée CRAC & the CT qui connecte les logiciels CRAC et CracTools afin d'identifier des séquences biomarqueurs répondant à une question biologique précise.

En outre, chacun des nœuds applicatifs 101 comporte une interface homme-machine 103 construite et agencée pour communiquer avec le système de calcul 102 du nœud applicatif 101. Cette interface homme-machine 103 peut être présentée sous la forme d'une application Web de type SaaS. Cette application WEB permet d'interfacer l'utilisateur avec un système de calcul 102 permettant d'organiser et d'interpréter les séquences biomarqueurs mais aussi de pouvoir consulter et éditer une base de connaissances.

Afin de pouvoir consulter et éditer la base de connaissances, l'architecture 100 comporte également une interface d'accès 104 à une base de connaissances 105, l'interface d'accès 104 à la base de connaissances 105 est par exemple formée par une API REST.

Le base de connaissances 105 est construite et agencée pour communiquer avec la pluralité de nœuds applicatifs 101 au moyen des interfaces d'accès 104. La base de connaissances 105 comporte notamment des données génomiques.

En outre, la base de connaissances 105 comporte une pluralité d'espaces privés 106, chacun des espaces privés 106 étant construit et agencé :
- pour contenir des métadonnées et des résultats issus d'un calcul réalisé par le système de calcul 102 d'une entité authentifiée comportant 1 à n utilisateur(s), et
- être accessible en lecture et en écriture uniquement aux utilisateurs de l'entité authentifiée.

Chaque entité comportant un ou plusieurs utilisateurs possède donc un espace privé 106 dans lequel les ressources (i.e. les résultats de calculs) sont cachées aux autres entités de l'architecture 100 dans le but de répondre au besoin de confidentialité que plusieurs utilisateurs souhaiteraient. Il n'est donc pas possible aux autres utilisateurs d'autres entités de les consulter.

Cette base privée 106 contient toutes les données de travail de l'utilisateur ou des utilisateurs d'une entité authentifiée. A cette fin, les données de l'espace privé 106 sont chiffrées par la clé de chiffrement de l'entité de l'utilisateur. Ainsi, seulement les utilisateurs de l'entité peuvent déchiffrer le contenu de leur espace privé 106 dans la base de connaissances 105.

La base de connaissances 105 comporte également une pluralité d'espaces partagés 107, chacun des espaces partagés 107 étant construit et agencé :
- pour contenir des métadonnées et des résultats issus de calculs collaboratifs réalisés par les systèmes de calcul 102 d'une entité authentifiée comportant 1 à n utilisateur(s), et
- être accessible en lecture et en écriture uniquement aux utilisateurs des entités authentifiés et bénéficiant d'un droit de consultation audit espace.

Chaque entité possède donc un espace partagé 107 pour lequel il est possible d'inviter d'autres utilisateurs d'une ou plusieurs autres entités afin d'effectuer un travail privé mais collaboratif. L'espace partagé 107 contient toutes les données communes dans le cadre d'un projet collaboratif entre plusieurs utilisateurs d'une ou plusieurs autres entités et ainsi permet des échanges de données entre utilisateurs appartenant à différentes entités mais travaillant sur un même projet.

Afin d'assurer la sécurité des données stockées, les données d'un espace partagé 107 sont stockées chiffrées dans la base de connaissances 105. Par conséquent, le stockage est accessible uniquement depuis les systèmes de calcul 102 authentifiés auprès de cet espace partagé 107.

Dans une mise en œuvre, l'utilisateur chiffre lui-même ses données dans la base de connaissances (commune) via l'interface homme-machine utilisée. Dans ce cas, la sécurité est augmentée par le fait que lui-seul a accès à sa clé de chiffrement et donc lui-seul est techniquement en mesure de lire les données chiffrées.

La base de connaissances 105 comporte également un espace commun 108, l'espace commun 108 étant accessible à tous les utilisateurs des entités authentifiées de l'architecture 100.

En d'autres termes, la base de connaissances 105 comporte une base publique 108 qui contient toutes les données publiques et accessibles par tous les utilisateurs enregistrés dans l'architecture 100. Il convient de souligner que tout tiers non authentifié auprès de l'architecture 100 n'a pas accès à cet espace commun 108, ni même d'ailleurs à l'architecture 100.

Il s'ensuit que l'architecture 100 offre la possibilité aux utilisateurs de regrouper les données en espace privé 106 (création d'un groupe par affinité), en espace partagé 107 (création d'un groupe de taille plus importante par affinité) ou en espace commun 108. Un espace 106, 107 ou 108 constitue un ensemble de données rassemblées selon un ou plusieurs critères.

Un espace 106, 107 ou 108 peut constituer, par exemple, un ensemble de données rassemblées selon un critère de confidentialité (privé, partagé ou commun) et de propriété (le groupe d'utilisateurs ayant insérés ces données).

En outre, un utilisateur appartenant à une entité peut, à tout moment, déplacer au moyen de son interface homme-machine 103 ses données d'un espace vers un autre au sein de la base de connaissances 105. Il a ainsi le contrôle total de ses données et peut les partager, à tout instant, avec le réseau d'utilisateurs de son choix. Il ne peut modifier que le critère de confidentialité, pas le critère de propriété.

Par exemple, un utilisateur A partage certaines de ses données pour qu'elles soient accessibles par un utilisateur B. Il déplace alors ses données de son espace privé 106 vers un espace partagé 107 de son entité en utilisant sa clé de déchiffrement, puis il associe un droit de lecture à cet espace partagé 107 à l'utilisateur B. Ce dernier peut donc consulter les séquences biomarqueurs présentes dans la base partagée 107 de l'utilisateur A.

Selon un autre exemple, un utilisateur C partage certaines de ses données pour qu'elles soient accessibles par tous les utilisateurs enregistrés dans l'architecture 100. Il déplace alors ses données de son espace privé 106, au moyen de son interface homme-machine 103, vers l'espace commun 108 en utilisant sa clé de déchiffrement. Tous les utilisateurs authentifiés auprès de l'architecture 100 ont alors accès aux données déchiffrées.

Dans une réalisation non limitative, les séquences biomarqueurs de la base de connaissances sont toutes associées à des mots clés. Si un utilisateur travaille sur un type particulier de données, il peut partager son travail en utilisant les mots clés associés. Dans ce cas, les autres utilisateurs travaillant sur ce type particulier de données en seront immédiatement informé, et l'association est soit commune, soit privée.

Par ailleurs, comme déjà évoqué, dès lors qu'un calcul effectué par un utilisateur via au moins un système de calcul 102 est terminé, les résultats sont transmis à la base de connaissances 105 via une interface d'accès 104 qui permet de relier du code logiciel à la base de connaissances 105, par exemple via une interface de type API REST. Le résultat de chaque calcul correspond à une liste de séquences biomarqueurs de type différent en fonction du logiciel de bio-informatique implémenté.

L'interface homme-machine 103 permet à l'utilisateur de déposer des fichiers de données NGS et d'interfacer l'utilisateur avec un système de calcul 102 permettant d'organiser et d'interpréter les séquences biomarqueurs, mais aussi de pouvoir consulter et éditer la base de connaissances 105.

Cette base de connaissances 105 est ainsi capable de stocker des informations de différents types, de pouvoir connecter toutes ces informations entre-elles et d'associer des métadonnées aux séquences biomarqueurs, puis de les partager entre plusieurs utilisateurs avec un niveau de sécurité préalablement défini.

En d'autres termes, cette base de connaissances 105 comporte des données publiques et est continuellement alimentée par les calculs (ou analyses) des utilisateurs, puis corrigée et mise à jour. En effet, chaque utilisateur peut, à la fois, consolider ses résultats par rapport à l'ensemble des biomarqueurs qu'il aura identifié, puis compléter à son tour le contenu de la base de connaissances en associant des nouvelles métadonnées à des biomarqueurs.

Par exemple, un clinicien peut échanger, pour chaque patient ou protocole clinique, ses informations et interprétations avec d'autres centres (ex. réseau entre plusieurs centre hospitalier universitaire), ce qui lui permettra à son tour d'enrichir les données sur la pathologie du patient, mieux comprendre et prendre en charge le traitement, et ainsi pouvoir intégrer les informations répertoriées dans la base de connaissances 105.

Cette architecture 100 modulaire offre une souplesse permettant de s'intégrer facilement aux futurs outils médicaux ce qui permettra de compléter facilement le panel d'offres avec de nouvelles applications mais aussi de garantir les résultats les plus précis du marché. Ce type d'architecture 100 permet à l'utilisateur d'accéder directement aux applications via l'interface homme-machine 103 et de consulter ou télécharger ses résultats interprétés en ligne.

Dans une mise en œuvre non limitative, l'architecture 100 comporte un ensemble de ressources 109 permettant d'organiser les séquences biomarqueurs présentes dans la base de connaissances 105.

Ces ressources sont toutes implémentées au sein de la base de connaissances 105.

Par exemple, l'architecture 100 comporte un système d'identification d'une entité et des éléments qu'elle manipule (par exemple des reads). Comme précédemment évoqué, une entité est formée par un regroupement d'utilisateurs et des éléments qu'ils manipulent. Par conséquent, l'entité peut désigner un ou plusieurs utilisateur(s) mais peut aussi identifier un service de l'utilisateur ou des utilisateur(s) (dans ce dernier cas, ce service est totalement indépendant des autres services de ce même utilisateur).

Par exemple, une analyse possède son propre identifiant mais également des informations de propriété (utilisateur et entité) et de confidentialité (espace commun, partagé ou privé).

L'architecture 100 comporte aussi un système d'identification et d'authentification d'un utilisateur. Par conséquent, le système d'identification et d'authentification d'un utilisateur représente un utilisateur de l'architecture, par exemple un chercheur ou encore un clinicien. En d'autres termes, une personne qui crée et consulte les données issues d'un séquençage à très haut débit, n'est en aucun cas un patient analysé. L'utilisateur est identifié avec un nom unique (le login) et s'authentifie avec un mot de passe que lui seul connaît. Le système identifie l'utilisateur si ce dit utilisateur existe dans la base de données d'identification. Le système utilise un « digest » (somme de contrôle du mot de passe) de la donnée d'authentification pour effectuer une comparaison et valider l'authentification. La base de données d'identification contient pour chaque utilisateur la clé de chiffrement de son entité de rattachement. Cette clé est elle-même chiffrée avec le mot de passe de l'utilisateur. Pendant l'authentification, le système utilise donc le mot de passe de l'utilisateur pour libérer la clé de chiffrement de l'entité. Cette clé est ensuite chiffrée avec un système asymétrique avant d'être renvoyée à l'utilisateur. Avec cette architecture, on obtient ces fonctionnalités de sécurité :
- les mots de passe ne sont pas stockés,
- les clés de chiffrements ne sont accessibles qu'avec un mot de passe valide d'un utilisateur autorisé,
- l'utilisateur ne peut pas consulter sa propre clé de chiffrement,
- l'utilisateur transporte avec lui un certificat assurant qu'il a bien été identifié et authentifié.

L'architecture 100 peut également comporter un système d'identification d'un calcul associé à au moins un utilisateur et une entité. En d'autres termes, ce système permet de désigner les résultats d'une analyse associée de façon unique à un utilisateur qui est l'initiateur de l'analyse ainsi qu'à l'entité reliée à l'initiateur de l'analyse. Par exemple, le système d'identification d'un calcul associé à au moins un utilisateur et une entité peut contenir des informations obtenues avec la suite logicielle CRAC & the CT. Les données résultantes peuvent être utilisées pour la détection de séquences biomarqueurs et alimentent la base de connaissances.

L'architecture 100 peut également comporter un système permettant de corréler des résultats obtenus.

Par exemple, si une analyse génère un biomarqueur erroné biologiquement, il est possible de rechercher les analyses ayant eu le même résultat précédent et ainsi éviter des tests biologiques inutiles.

L'architecture 100 peut également comporter un système de regroupement logique d'identifiants de calculs associés à un utilisateur et une entité. Ce système de regroupement permet d'améliorer la compréhension d'un problème biologique, le pronostic ou le diagnostic d'un patient puisqu'il permet d'avoir une vision d'ensemble des calculs (ex. les séquences biomarqueurs) qui sont corrélés entre eux en appliquant une règle définie.

Par exemple, il est possible de regrouper les analyses issues de patients atteint d'une même pathologie pour en déterminer des points communs ou des informations statistiques.

L'architecture 100 peut également comporter un système permettant d'associer une méta-information à des données de la base de connaissances. Ce système peut être utilisé par les utilisateurs en vue d'associer un message texte commentant le résultat d'un calcul. Par exemple, il est possible de générer une discussion inter-entité sur une problématique identifiée par un groupe d'analyse ou un marqueur spécifique. Cette discussion permet un échange rapide parmi les différents spécialistes sur des questions précises.

Dans une mise en œuvre non limitative, la base de connaissances 105 est formée par un anneau de base de données.

En d'autres termes, le stockage des données repose sur une base de connaissances en anneau de base de données de type clé/colonne. Ainsi, tous les utilisateurs ont accès en temps réel à la base de connaissances en lecture et en écriture. La base de connaissances est constituée de ressources pouvant être ajoutées/supprimées en fonction du besoin de capacité et de performance, agrandissant ainsi l'anneau de stockage. Cela permet de tirer avantage d'un stockage distribué, illimité et hautement disponible puisque les données sont répliquées à trois endroits différents.

L'emplacement de ces réplications est automatiquement calculé selon des critères (machine et géographique) de sorte qu'il couvre l'ensemble des pannes possibles. Par exemple. une donnée A est stockée sur une machine X avec deux autres copies sur les machines Y et Z. Toutes les machines X,Y et Z constituent l'intégralité de la base de connaissances.

On rappelle que lorsque l'on prête une action à la base de connaissances, celle-ci est en fait effectuée par un microprocesseur de la base de connaissances commandé par des codes instructions enregistrés dans une mémoire de la base de connaissances. Si l'on prête une action à une application, celle-ci est en fait effectuée par un microprocesseur de la base de connaissances dans une mémoire duquel les codes instructions correspondant à l'application sont enregistrés.

De manière générale, dans une base de données classique, existe le théorème du «CAP » : le C étant mis pour « Consistency » (cohérence), le A pour « Availability » (disponibilité) et le P pour « Partition » (tolérance aux pannes et capacité). Selon l'invention, la base de connaissances est axée sur le A pour toujours garantir une réponse et sur le P pour permettre un stockage massif (« big data »). Quant au C, il est suffisamment optimal pour répondre aux besoins de cohérence dans le cadre de l'utilisation. Aucune base de données actuelle ne peut remplir les trois conditions (CAP). À l'architecture selon l'invention, il est ajouté en outre un second C pour « Confidentiality » (confidentialité) car elle permet de garantir un niveau de sécurité plus important que la norme en vigueur et qu'une base de données classique pourrait remplir.

En outre, dans la mise en œuvre de l'architecture selon l'invention, pour chaque analyse (ou calcul) sont générés des éléments de la base de connaissances (biomarqueurs génomiques). Chacun de ces éléments contient une référence sur la ou les analyses qui l'ont générée. De ce fait, il est possible de regrouper les analyses partageant les mêmes éléments (biomarqueurs) et, par extrapolation, les utilisateurs des analyses peuvent être mis en relation et peuvent se mettre eux-mêmes en relation.

L'architecture selon l'invention permet de démocratiser complètement l'analyse pour qu'un clinicien ou un biologiste soit complètement autonome sur son analyse (plus besoin de spécialistes en bio-informatique et bio-statistique entre lui et sa donnée NGS), avec un niveau de sécurité choisi, un partage défini, une qualité garantie, des outils visuels adaptés et un partage délimité.

## Revendications

1. Architecture (100) pour l'analyse de données génomiques comportant :
- une pluralité de nœuds applicatifs (101), chacun des nœuds applicatifs comportant :
- un système de calcul (102) comportant au moins un nœud de calcul, le système de calcul (102) étant construit et agencé pour réaliser des calculs de génomique,
- une interface homme-machine (103) construite et agencée pour communiquer avec le système de calcul (102),
- une interface d'accès (104) à une base de connaissances (105),
- une base de connaissances (105) construite et agencée pour communiquer avec la pluralité de nœuds applicatifs (101), ladite base de connaissances (105) contenant des données génomiques;
- une pluralité d'espaces privés (106) contenus dans la base de connaissances (105), chacun des espaces privés (106) étant construit et agencé :
- pour contenir des métadonnées et des résultats issus d'un calcul réalisé par le système de calcul (102) d'une entité authentifiée comportant 1 à n utilisateur(s),
**caractérisée en ce que** lesdits résultats étant chiffrés par une clé de chiffrement de ladite entité, et
- pour être accessible en lecture uniquement aux utilisateurs de l'entité authentifiée ;
- un système d'identification et d'authentification d'un utilisateur,
le système d'identification et d'authentification étant configuré :
- pour identifier ledit utilisateur dans une base de données d'identification contenant pour chaque utilisateur la clé de chiffrement de l'entité correspondant audit utilisateur,
- pour utiliser un mot de passe connu uniquement de l'utilisateur pour libérer ladite clé de chiffrement de ladite entité correspondant audit utilisateur et renvoyer ladite clé de chiffrement chiffrée avec un système asymétrique audit utilisateur.

2. Architecture (100) pour l'analyse de données génomiques selon la revendication 1, ladite architecture (100) étant **caractérisée en ce qu'**elle comporte une pluralité d'espaces partagés 107, chacun des espaces partagés (107) étant construit et agencé :
pour contenir des métadonnées et des résultats issus de calculs collaboratifs réalisés par les systèmes de calcul (102) d'une entité authentifiée comportant 1 à n utilisateur(s), et
être accessible en lecture uniquement aux utilisateurs des entités authentifiés et bénéficiant d'un droit de consultation audit espace partagé (107).

3. Architecture (100) pour l'analyse de données génomiques selon l'une quelconque des revendications précédente, ladite architecture (100) étant **caractérisée en ce qu'**elle comporte un espace commun (108), ledit espace commun (108) étant accessible à tous les utilisateurs des entités authentifiés de l'architecture (100).

4. Architecture (100) selon l'une quelconque des revendications précédentes, ladite architecture (100) étant **caractérisée en ce que** le système de calcul (102) est construit et agencé pour classer des courtes séquences numériques de matériel biologique

5. Architecture (100) selon la revendication 4 précédente, ladite architecture (100) étant **caractérisée en ce que** le système de calcul (102) est construit et agencé pour agréger les courtes séquences numériques de matériel biologique d'une même classe.

6. Architecture (100) selon l'une quelconque des revendications précédentes, ladite architecture (100) étant **caractérisée en ce que** le système de calcul (102) est construit et agencé pour identifier une séquence biomarqueur répondant à une question biologique

7. Architecture (100) selon l'une quelconque des revendications précédentes, ladite architecture (100) étant **caractérisée en ce qu'**elle comporte une pluralité de ressources (109), lesdites ressources (109) étant formées par :
- Un système d'identification d'une entité et des éléments qu'elle manipule,
- Un système d'identification d'un utilisateur,
- Un système d'identification d'un calcul associé à au moins un utilisateur et une entité,
- Un système permettant de corréler des calculs réalisés,
- Un système de regroupement logique d'identifiants de calculs associés à un utilisateur et une entité,
- Un système permettant d'associer une méta-information à des données de la base de connaissances (105).

8. Architecture (100) selon l'une quelconque des revendications précédentes, ladite architecture (100) étant **caractérisée en ce que** la base de connaissance (105) est formée par un anneau de base de données.

9. Architecture (100) selon l'une quelconque des revendications précédentes, ladite architecture (100) étant **caractérisée en ce que** l'interface d'accès (104) à la base de connaissances (105) est une API REST.

## Patentansprüche

1. Architektur (100) für die Analyse von Genomdaten, die Folgendes umfasst:
- eine Vielzahl von Anwendungsknoten (101), wobei jeder der Anwendungsknoten Folgendes umfasst:
- ein Berechnungssystem (102), das mindestens einen Berechnungsknoten umfasst, wobei das Berechnungssystem (102) gebaut und angeordnet ist, um Genomikberechnungen durchzuführen,
- eine Mensch-Maschine-Schnittstelle (103), die für die Kommunikation mit dem Berechnungssystem (102) gebaut und angeordnet ist,
- eine Schnittstelle (104) für den Zugriff auf eine Wissensdatenbank (105),
- eine Wissensdatenbank (105), die so gebaut und angeordnet ist, dass sie mit der Vielzahl von Anwendungsknoten (101) kommuniziert, wobei die Wissensdatenbank (105) Genomdaten enthält;
- eine Vielzahl von privaten Räumen (106), die in der Wissensdatenbank (105) enthalten sind, wobei jeder der privaten Räume (106) für Folgendes gebaut und angeordnet ist:
- zum Erhalten von Metadaten und Ergebnissen aus einer Berechnung, die vom Berechnungssystem (102) einer authentifizierten Einheit mit 1 bis n Benutzer(n) durchgeführt wurde, **dadurch gekennzeichnet, dass** die Ergebnisse durch einen Chiffrierschlüssel der Einheit verschlüsselt sind, und
- zum Lesen nur für Benutzer der authentifizierten Entität zugänglich sein;
- ein System zur Identifizierung und Authentifizierung eines Benutzers,
wobei das Authentifizierungs- und Identifizierungssystem für Folgendes eingerichtet :
- zur Identifizierung des Benutzers in einer Identifizierungsdatenbank, die für jeden Benutzer den Chiffrierschlüssel der Entität enthält, die diesem Benutzer entspricht,
- zum Verwenden eines Passworts, das nur dem Benutzer bekannt ist, um den Chiffrierschlüssel der Entität freizugeben, die diesem Benutzer entspricht, und den Chiffrierschlüssel mit einem asymmetrischen System an diesen Benutzer zurückzusenden.

2. Architektur (100) für die Analyse von Genomdaten nach Anspruch 1, wobei die Architektur (100) **dadurch gekennzeichnet ist, dass** sie eine Vielzahl von gemeinsamen Räumen (107) umfasst, wobei jeder der gemeinsamen Räume (107) für Folgendes gebaut und angeordnet ist:
zum Erhalten von Metadaten und Ergebnissen aus kollaborativen Berechnungen, die von den Berechnungssystemen (102) einer authentifizierten Einheit mit 1 bis n Benutzer(n) durchgeführt werden, und
schreibgeschützt für die Benutzer der authentifizierten Einheiten zugänglich sein, die über ein Zugriffsrecht auf diesen gemeinsam genutzten Bereich verfügen (107).

3. Architektur (100) zur Analyse von Genomdaten nach einem der vorhergehenden Ansprüche, wobei die Architektur (100) **dadurch gekennzeichnet ist, dass** sie einen gemeinsamen Raum (108) umfasst, wobei der gemeinsame Raum (108) für alle Benutzer der authentifizierten Objekte der Architektur (100) zugänglich ist.

4. Architektur (100) nach einem der vorhergehenden Ansprüche, wobei die Architektur (100) **dadurch gekennzeichnet ist, dass** das Berechnungssystem (102) so gebaut und angeordnet ist, dass es kurze numerische Sequenzen von biologischem Material klassifiziert

5. Architektur (100) nach dem vorhergehenden Anspruch 4, wobei die Architektur (100) **dadurch gekennzeichnet ist, dass** das Berechnungssystem (102) so aufgebaut und angeordnet ist, dass es die kurzen numerischen Sequenzen von biologischem Material derselben Klasse aggregiert.

6. Architektur (100) nach einem der vorhergehenden Ansprüche, wobei die Architektur (100) **dadurch gekennzeichnet ist, dass** das Berechnungssystem (102) gebaut und angeordnet ist, um eine Biomarkersequenz zu identifizieren, die eine biologische Frage beantwortet

7. Architektur (100) nach einem der vorhergehenden Ansprüche, wobei die Architektur (100) **dadurch gekennzeichnet ist, dass** sie eine Vielzahl von Ressourcen (109) umfasst, wobei die Ressourcen (109) aus Folgendem gebildet werden:
- Einem System zur Identifizierung einer Einheit und der Elemente, mit denen sie arbeitet,
- Einem System zur Identifizierung eines Benutzers,
- Einem System zur Identifizierung einer Berechnung, die mindestens einem Benutzer und einer Einheit zugeordnet ist,
- Einem System zur Korrelation der durchgeführten Berechnungen,
- Einem System zur logischen Gruppierung von Berechnungskennungen, die einem Benutzer und einer Einheit zugeordnet sind,
- Einem System, mit dem eine Metainformation mit Daten aus der Wissensdatenbank (105) verknüpft werden kann.

8. Architektur (100) nach einem der vorhergehenden Ansprüche, wobei die Architektur (100) **dadurch gekennzeichnet ist, dass** die Wissensbasis (105) durch einen Datenbankring gebildet ist.

9. Architektur (100) nach einem der vorhergehenden Ansprüche, wobei die Architektur (100) **dadurch gekennzeichnet ist, dass** die Schnittstelle (104) für den Zugriff auf die Wissensdatenbank (105) eine REST-API ist.

## Claims

1. Architecture (100) for genomic data analysis comprising:
- a plurality of applicative nodes (101), each applicative node comprising:
- a computing system (102) comprising at least one computing node, the computing system (102) being constructed and configured to perform genomic computations,
- a human-machine interface (103) constructed and configured to communicate with the computing system (102),
- an access interface (104) to a knowledge base (105),
- a knowledge base (105) constructed and configured to communicate with the plurality of applicative nodes (101), said knowledge base (105) containing genomic data;
- a plurality of private spaces (106) contained within the knowledge base (105), each private space (106) being constructed and configured:
- to contain metadata and results from a computation performed by the computing system (102) of an authenticated entity comprising 1 to n user(s), **characterized in that** said results are encrypted using an encryption key of said entity, and
- to be accessible in read-mode only to users of the authenticated entity;
- an identification and authentication system for a user, the identification and authentication system being configured:
- to identify said user in an identification database containing for each user the encryption key of the entity corresponding to said user,
- to use a password known only to the user to release said encryption key of said entity corresponding to said user and return said encryption key encrypted with an asymmetric system to said user.

2. Architecture (100) for genomic data analysis according to claim 1, said architecture (100) being **characterized in that** it comprises a plurality of shared spaces (107), each shared space (107) being constructed and configured:
to contain metadata and results from collaborative computations performed by the computing systems (102) of an authenticated entity comprising 1 to n user(s), and
to be accessible in read-mode only to users of authenticated entities and benefiting from a right of consultation rights of said shared space (107).

3. Architecture (100) for genomic data analysis according to any one of the preceding claims, said architecture (100) being **characterized in that** it comprises a common space (108), said common space (108) being accessible to all users of the authenticated entities of the architecture (100).

4. Architecture (100) according to any one of the preceding claims, said architecture (100) being **characterized in that** the computing system (102) is constructed and configured to classify short numerical sequences of biological material.

5. Architecture (100) according to the preceding claim 4, said architecture (100) being **characterized in that** the computing system (102) is constructed and configured to aggregate said short numerical sequences of biological material of a same class.

6. Architecture (100) according to any one of the preceding claims, said architecture (100) being **characterized in that** the computing system (102) is constructed and configured to identify a biomarker sequence responding to a biological question.

7. Architecture (100) according to any one of the preceding claims, said architecture (100) being **characterized in that** it comprises a plurality of resources (109), said resources (109) being formed by:
- An identification system of an entity and elements it manipulates;
- An identification system of a user,
- An identification system of a computation associated to at least one user and one entity,
- A system allowing to correlate performed computations,
- A logical grouping system of computation identifiers associated to a user and an entity,
- A system allowing to associate a meta-information to data of the knowledge base (105).

8. Architecture (100) according to any one of the preceding claims, said architecture (100) being **characterized in that** the knowledge base (105) is formed by a database ring.

9. Architecture (100) according to any one of the preceding claims, said architecture (100) being **characterized in that** the access interface (104) to the knowledge base (105) is a REST API.
